# EUROPÄISCHE PATENTSCHRIFT

(11) **EP 1 479 432 B1**
(45) Veröffentlichungstag und Bekanntmachung des Hinweises auf die Patenterteilung: **27.06.2007**
(21) Anmeldenummer: 03011398.9
(22) Anmeldetag: 20.05.2003
(51) Int. Cl.: B01J 13/02, B01J 13/22

(54) **Mikrokapseln (XXIV)**
Microcapsules (XXIV)
Microcapsules (XXIV)

(43) Veröffentlichungstag der Anmeldung: 24.11.2004
(73) Patentinhaber: Cognis IP Management GmbH, 40589 Düsseldorf (DE)
(72) Erfinder: Viladot Petit, Josep-Lluis, Dr., 08018 Barcelona (ES); Asensio, Juan-Antonio, 08820 El Prat de Llobregat (Barcelona) (ES); Alaoui Ismaili, Smail, 08290 Cerdanyola del Vallès (ES); Rull Prous, Santiago, 08034 Barcelona (ES); Escudero, Joaquin, 08015 Barcelona (ES); Fabry, Bernd, Dr., 41352 Korschenbroich (DE)

(56) Entgegenhaltungen:
- EP-A- 1 101 527
- EP-A- 1 247 568
- US-A- 3 748 277
- US-A- 5 985 354

## Beschreibung

### Gebiet der Erfindung

Die Erfindung befindet sich auf dem Gebiet der verkapselten Wirkstoffe und betrifft neue Mikrokapseln mit doppelter Hüllmembran, ein Verfahren zu deren Herstellung sowie ihre Verwendung in einer Reihe von Anwendungsgebieten.

### Stand der Technik

Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

In diesem Zusammenhang sei auf die europäische Patentanmeldung EP 1101527 A1 (Cognis) sowie die internationale Patentanmeldung WO 01/28530 (Lipotec) verwiesen, aus der mikroverkapselte Wirkstoffe, insbesondere auch Pflanzenextrakte bekannt sind. Die Matrix dieser Kapseln besteht im wesentlichen aus Stärke, während die Hülle von Chitosan gebildet wird. Von Nachteil hat sich jedoch gezeigt, dass die Kapseln nicht immer über eine ausreichende chemische Beständigkeit besitzen. Insbesondere bei Einsatz bestimmter Pflanzenextrakte kann es zu Wechselwirkungen mit der Hüllsubstanz kommen, wodurch die Membran spontan permeabel wird und die Wirkstoffe freisetzt.

Die Aufgabe der vorliegenden Erfindung hat somit darin bestanden, neue Mikrokapseln zur Verfügung zu stellen, die gegenüber den bekannten Kapseln des Standes der Technik über eine verbesserte chemische Beständigkeit und Bioverfügbarkeit verfügen und die darin enthaltenen Wirkstoffe verzögert freisetzen. Eine zusätzliche Aufgabe hat ferner darin bestanden, auch die Verkapselung von Wirkstoffen mit starkem Eigengeruch, wie z.B. Valerianextrakt oder Lipoinsäure, sicherzustellen, so dass die Mikrokapseln selbst geruchsfrei sind.

### Beschreibung der Erfindung

Gegenstand der Erfindung sind Mikrokapseln mit mittleren Durchmessern im Bereich von 0,001 bis 0,5, vorzugsweise 0,01 bis 0,1 mm, bestehend aus einer doppelten Hüllmembran und einer die Wirkstoffe enthaltenden Matrix, dadurch erhältlich, dass man
(a) eine Lösung mindestens eines Wirkstoffs in einem geeigneten Lösungsmittel herstellt,
(b) die Lösung mit einem Polysaccharid versetzt,
(c) die so erhaltene Matrix bei pH-Werten im Bereich von 6 bis 8 mit einer Chitosanlösung versetzt,
(d) die so erhaltenen Mikrokapseln vom Lösungsmittel befreit und
(e) mit einer Lösung eines filmbildenden nichtionischen Polymers behandelt.

Überraschenderweise wurde gefunden, dass durch die Verwendungkationischen Biopolymeren vom Typ des Chitosans und nichtionischen Polymeren Mikrokapseln mit einer doppelten Hülle erhalten werden, die das vorgegebene Anforderungsprofil zur Zufriedenheit erfüllen. Zum einen werden auch dann geruchsfreie Kapseln erhalten, wenn Wirkstoffe mit starkem Eigengeruch verkapselt werden, zum anderen kommt es auch bei Verkapselung beliebiger pflanzlicher Extrakte nicht mehr zu Wechselwirkungen mit der Hüllsubstanz und zur ungewünschten spontanen Freisetzung der Wirkstoffe.

Ein weiterer Gegenstand der vorliegenden Erfindung betrifft ein Verfahren zur Herstellung von Mikrokapseln mit mittleren Durchmessern im Bereich von 0,001 bis 0,5, vorzugsweise 0,01 bis 0,1 mm, bestehend aus einer doppelten Hüllmembran und einer die Wirkstoffe enthaltenden Matrix, bei dem man
(a) eine Lösung mindestens eines Wirkstoffs in einem geeigneten Lösungsmittel herstellt,
(b) die Lösung mit einem Polysaccharid versetzt,
(c) die so erhaltene Matrix bei pH-Werten im Bereich von 6 bis 8 mit einer Chitosanlösung versetzt,
(d) die so erhaltenen Mikrokapseln vom Lösungsmittel befreit und
(e) mit einer Lösung eines filmbildenden nichtionischen Polymers behandelt.

### Wirkstoffe

Die Auswahl der im Sinne der Erfindung in Betracht kommenden Wirkstoffen ist wenig kritisch und richtet sich in erster Linie danach, in welchem Anwendungsgebiet die Mikrokapseln eingesetzt werden sollen. Für Anwendungen in den Bereichen Kosmetik, Pharmazie, Ernährung und Textiltechnik kommen beispielsweise Tocopherol, Tocopherolacetat, Tocopherolpalmitat, Ascorbinsäure, (Desoxy)Ribonucleinsäuren, Retinol, Bisabolol, Allantoin, Phytantriol, Panthenol, AHA-Säuren, Kojisäure, Lipoinsäure, Aminosäuren, Ceramiden, Pseudoceramiden, essentiellen Ölen, Pflanzenextrakten und Vitaminkomplexen sowie deren Gemischen in Frage. Werden die Mikrokapseln im Agrosektor eingesetzt, kommen als Wirkstoffe auch Pestizide in Frage. Der Anteil der Wirkstoffe an den Mikrokapseln kann bezogen auf das Trockengewicht der Kapseln 1 bis 30, vorzugsweise 5 bis 25 Gew.-% betragen.

### Herstellung der Matrix

Zur Herstellung der Matrix wird im ersten Schritt eine Lösung oder Dispersion der Wirkstoffe hergestellt, die dann mit den Polysacchariden oder deren Lösung versetzt wird. Als Polysaccharide eignen sich beispielsweise Stärke (Maisstärke, Weizenstärke, Reisstärke, Kartoffelstärke), Cellulose oder deren Derivate, insbesondere Carboxymethylcellulose. Wirkstoffe und Polysaccharide können im Gewichtsverhältnis 1 : 1 bis 10 : 1, vorzugsweise 1 : 2 bis 1 : 5 eingesetzt werden. Als Lösungsmittel kommen insbesondere Wasser, niedere aliphatische Alkohole oder deren Gemische in Frage. Besonders bevorzugt ist der Einsatz von wässrigem Ethanol. Die Herstellung der Matrix kann bei Raumtemperatur oder erhöhten Temperaturen von 25 bis 50 °C erfolgen, wobei es sich empfiehlt, die Komponenten unter starkem Rühren zu vermischen.

### Herstellung der Doppelhülle

Bei der Herstellung der Doppelhülle macht sich die Erfindung die Erkenntnis zu Nutze, dass Chitosane, die üblicherweise als saure Lösungen eingesetzt werden, im basischen Bereich koagulieren und Membranen bilden. Üblicherweise wird daher die Matrixlösung im zweiten Verfahrensschritt mit einer schwach sauren Lösung eines Chitosans bzw. Chitosansalzes behandelt und der pH-Wert durch Zugabe einer Base angehoben, vorzugsweise auf 7 bis 7,5, so dass die Koagulation erfolgt. Vorzugsweise werden solche Chitosantypen eingesetzt, die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt. Die Abtrennung des im wesentlichen wässrigen oder wässrig-alkoholischen Lösungsmittels kann bei schwacher Hitze unter vermindertem Druck erfolgen. Um eine homogene Größenverteilung zu gewährleisten, empfiehlt es sich, die Mikrokapseln nach dem Trocknungsschritt zu sieben. Im abschließenden Schritt erfolgt dann die Bildung der zweiten Hülle. Hierzu empfiehlt es sich, die getrockneten Mikrokapseln im Wirbelbett mit einer wässrigen oder gegebenenfalls alkoholischen Lösung des nichtionischen Polymers zu besprühen, um ein Zusammenbacken zu verhindern und eine gleichmäßige Bedeckung der Kapseln sicherzustellen. Unter den handelsüblichen nichtionischen Polymeren stellt Polyvinylpyrrolidon (PVP) das bevorzugte Einsatzmaterial dar.

### Gewerbliche Anwendbarkeit

Weitere Gegenstände der vorliegenden Erfindung betreffen die Verwendung der erfindungsgemäßen Mikrokapseln zur Herstellung von kosmetischen und/oder pharmazeutischen Zubereitungen, Nahrungsmittelergänzungsstoffen sowie von Mitteln für die Textiltechnik oder Landwirtschaft, in denen sie in Mengen von 1 bis 30, vorzugsweise 10 bis 20 Gew.-% enthalten sein können.

### Kosmetische und/oder pharmazeutische Zubereitungen

Die erfindungsgemäßen Mikrokapseln können insbesondere zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

### • Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### • Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### • Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### • Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### • Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care@ 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### • Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### • Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-snglycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminiumund/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat@L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
- Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate. Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

### Antioxidantien

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### • Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-ndecylamid.

### • Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder - phosphate, wie beispielsweise Lanosterin-, Cholesterin-, Campesterin-, Stigmasterinund Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### • Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

### • Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Stemanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

### Beispiel 1

In einer ersten Rührapparatur wurden 30 g des aktiven Wirkstoffs Valerianextrakt in 70 ml Ethanol gelöst, in einer zweiten Apparatur 45 g Maisstärke in 23 g Wasser suspendiert. Beide Lösungen wurden unter intensivem Rühren miteinander vermischt. Anschließend wurden 17,5 g einer 1 Gew.-%igen Lösung von Chitosan (Hydagen® CMF, Cognis) in Glycolsäure zugetropft. Durch Zugabe von wässriger Natriumhydroxidlösung wurde der pH-Wert von 7 bis 7,5 angehoben und das wässrige Ethanol im Vakuum abgetrennt. Die resultierenden Mikrokapseln wurden zur Homogenisierung der Größenverteilung gesiebt und dann in eine Wirbelschicht gebracht, wo sie im Gegenstrom heißer Luft mit einer wässrigen, 5 Gew.-%igen Lösung von PVP besprüht wurden.

### Beispiel 2

In einer ersten Rührapparatur wurden 3 g Retinol in 30 ml Ethanol gelöst, in einer zweiten Apparatur 45 g Kartoffelstärke in 23 g Wasser suspendiert. Beide Lösungen wurden unter intensivem Rühren miteinander vermischt. Anschließend wurden 17,5 g einer 1 Gew.-%igen Lösung von Chitosan (Hydagen® CMF, Cognis) in Glycolsäure zugetropft. Durch Zugabe von wässriger Natriumhydroxidlösung wurde der pH-Wert von 7 bis 7,5 angehoben und das wässrige Ethanol im Vakuum abgetrennt. Die resultierenden Mikrokapseln wurden zur Homogenisierung der Größenverteilung gesiebt und dann in eine Wirbelschicht gebracht, wo sie im Gegenstrom heißer Luft mit einer wässrigen, 5 Gew.-%igen Lösung von PVP besprüht wurden.

### Beispiel 3

In einer ersten Rührapparatur wurden 3 g Bisabolol in 30 ml Ethanol gelöst, in einer zweiten Apparatur 45 g Kartoffelstärke in 23 g Wasser suspendiert. Beide Lösungen wurden unter intensivem Rühren miteinander vermischt. Anschließend wurden 17,5 g einer 1 Gew.-%igen Lösung von Chitosan (Hydagen® CMF, Cognis) in Glycolsäure zugetropft. Durch Zugabe von wässriger Natriumhydroxidlösung wurde der pH-Wert von 7 bis 7,5 angehoben und das wässrige Ethanol im Vakuum abgetrennt. Die resultierenden Mikrokapseln wurden zur Homogenisierung der Größenverteilung gesiebt und dann in eine Wirbelschicht gebracht, wo sie im Gegenstrom heißer Luft mit einer wässrigen, 5 Gew.-%igen Lösung von PVP besprüht wurden.

### Beispiel 4

In einer ersten Rührapparatur wurden 3 g gefriergetrockenter Extrakt von Grünem Tee in 30 ml Ethanol gelöst, in einer zweiten Apparatur 45 g Reisstärke in 23 g Wasser suspendiert. Beide Lösungen wurden unter intensivem Rühren miteinander vermischt. Anschließend wurden 17,5 g einer 1 Gew.-%igen Lösung von Chitosan (Hydagen® CMF, Cognis) in Glycolsäure zugetropft. Durch Zugabe von wässriger Natriumhydroxidlösung wurde der pH-Wert von 7 bis 7,5 angehoben und das wässrige Ethanol im Vakuum abgetrennt. Die resultierenden Mikrokapseln wurden zur Homogenisierung der Größenverteilung gesiebt und dann in eine Wirbelschicht gebracht, wo sie im Gegenstrom heißer Luft mit einer wässrigen, 5 Gew.-%igen Lösung von PVP besprüht wurden.

### Beispiel 5

In einer ersten Rührapparatur wurden 3 g Ribonucleinsäure in 30 ml Ethanol gelöst, in einer zweiten Apparatur 45 g Carboxymethylcellulose in 23 g Wasser suspendiert. Beide Lösungen wurden unter intensivem Rühren miteinander vermischt. Anschließend wurden 17,5 g einer 1 Gew.-%igen Lösung von Chitosan (Hydagen® CMF, Cognis) in Glycolsäure zugetropft. Durch Zugabe von wässriger Natriumhydroxidlösung wurde der pH-Wert von 7 bis 7,5 angehoben und das wässrige Ethanol im Vakuum abgetrennt. Die resultierenden Mikrokapseln wurden zur Homogenisierung der Größenverteilung gesiebt und dann in eine Wirbelschicht gebracht, wo sie im Gegenstrom heißer Luft mit einer wässrigen, 5 Gew.-%igen Lösung von PVP besprüht wurden.

### Beispiel 6

In einer ersten Rührapparatur wurden 3 g Lipoinsäure in 30 ml Ethanol gelöst, in einer zweiten Apparatur 45 g Maisstärke in 23 g Wasser suspendiert. Beide Lösungen wurden unter intensivem Rühren miteinander vermischt. Anschließend wurden 17,5 g einer 1 Gew.-%igen Lösung von Chitosan (Hydagen® CMF, Cognis) in Glycolsäure zugetropft. Durch Zugabe von wässriger Natriumhydroxidlösung wurde der pH-Wert von 7 bis 7,5 angehoben und das wässrige Ethanol im Vakuum abgetrennt. Die resultierenden Mikrokapseln wurden zur Homogenisierung der Größenverteilung gesiebt und dann in eine Wirbelschicht gebracht, wo sie im Gegenstrom heißer Luft mit einer wässrigen, 5 Gew.-%igen Lösung von PVP besprüht wurden.

**Tabelle 1**

| **Beispiele für kosmetische Zubereitungen (Wasser, Konservierungsmittel ad 100 Gew.-%)** | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Texapon® NSO** Sodium Laureth Sulfate | - | - | - | - | - | - | 38,0 | 38,0 | 25,0 | - |
| **Texapon® SB 3** Disodium Laureth Sulfosuccinate | - | - | - | - | - | - | - | - | 10,0 | - |
| **Plantacare® 818** Coco Glucosides | - | - | - | - | - | - | 7,0 | 7,0 | 6,0 | - |
| **Plantacare® PS 10** Sodium Laureth Sulfate (and) Coco Glucosides | - | - | - | - | - | - | - | - | - | 16,0 |
| **Dehyton® PK 45** Cocamidopropyl Betaine | - | - | - | - | - | - | - | - | 10,0 | - |
| **Dehyquart® A** Cetrimonium Chloride | 2,0 | 2,0 | 2,0 | 2,0 | 4,0 | 4,0 | - | - | - | - |
| **Dehyquart L® 80** Dicocoylmethylethoxymonium Methosulfate (and) Propylenglycol | 1,2 | 1,2 | 1,2 | 1,2 | 0,6 | 0,6 | - | - | - | - |
| **Eumulgin® B2** Ceteareth-20 | 0,8 | 0,8 | - | 0,8 | - | 1,0 | - | - | - | - |
| **Eumulgin® VL 75** Lauryl Glucoside (and) Polyglyceryl-2 Polyhydroxystearate (and) Glycerin | - | - | 0,8 | - | 0.8 | - | - | - | - | - |
| **Lanette® O** Cetearyl Alcohol | 2,5 | 2,5 | 2,5 | 2,5 | 3,0 | 2,5 | - | - | - | - |
| **Cutina® GMS** Glyceryl Stearate | 0,5 | 0,5 | 0,5 | 0,5 | 0,5 | 1,0 | - | - | - | - |
| **Cetiol® HE** PEG-7 Glyceryl Cocoate | 1,0 | - | - | - | - | - | - | - | 1,0 | |
| **Cetiol® PGL** Hexyldecanol (and) Hexyldecyl Laurate | - | 1,0 | - | - | 1,0 | - | - | - | - | - |
| **Cetiol® V** Decyl Oleate | - | - | - | 1,0 | - | - | - | - | - | - |
| **Eutanol® G** Octyldodecanol | - | - | 1,0 | - | - | 1,0 | - | - | - | - |
| **Nutrilan® Keratin W** Hydrolyzed Keratin | - | - | - | 2,0 | - | - | - | - | - | - |
| **Lamesoft® LMG** Glyceryl Laurate (and) Potassium Cocoyl Hydrolyzed Collagen | - | - | - | - | - | - | 3,0 | 2,0 | 4,0 | - |
| **Euperlan® PK 3000 AM** Glycol Distearate (and) Laureth-4 (and) Cocamidopropyl Betaine | - | - | - | - | - | - | - | 3,0 | 5,0 | 5,0 |
| **Generol® 122 N** Soja Sterol | - | - | - | - | 1,0 | 1,0 | - | - | - | - |
| **Mikrokapseln nach Beispiel 4** | 1,0 | 1,0 | 1,0 | 1,0 | 1,0 | 1,0 | 1,0 | 1,0 | 1,0 | 1,0 |
| **Hydagen® CMF** Chitosan | 1,0 | 1,0 | 1,0 | 1,0 | 1,0 | 1,0 | 1,0 | 1,0 | 1,0 | 1,0 |
| **Copherol® 1250** Tocopherol Acetate | - | - | 0,1 | 0,1 | - | - | - | - | - | - |
| **Arlypon® F** Laureth-2 | - | - | - | - | - | - | 3,0 | 3,0 | 1,0 | - |
| **Sodium Chloride** | - | - | - | - | - | - | - | 1,5 | - | 1,5 |

| **Zusammensetzung (INCI)** | **11** | **12** | **13** | **14** | **15** | **16** | **17** | **18** | **19** | **20** |
|---|---|---|---|---|---|---|---|---|---|---|
| **Texapon® NSO** Sodium Laureth Sulfate | 20,0 | 20,0 | 12,4 | - | 25,0 | 11,0 | - | - | - | - |
| **Texapon® K 14 S** Sodium Myreth Sulfate | - | - | - | - | - | - | - | - | 11,0 | 23,0 |
| **Texapon® SB 3** Disodium Laureth Sulfosuccinate | - | - | - | - | - | 7,0 | - | - | - | - |
| **Plantacare® 818** Coco Glucosides | 5,0 | 5,0 | 4,0 | - | - | - | - | - | 6,0 | 4,0 |
| **Plantacare® 2000** Decyl Glucoside | - | - | - | - | 5,0 | 4,0 | - | - | - | - |
| **Plantacare® PS 10** Sodium Laureth Sulfate (and) Coco Glucosides | - | - | - | 40,0 | - | - | 16,0 | 17,0 | - | - |
| **Dehyton® PK 45** Cocamidopropyl Betaine | 20,0 | 20,0 | - | - | 8,0 | - | - | - | - | 7,0 |
| **Eumulgin® B1** Ceteareth-12 | - | - | - | - | 1,0 | - | - | - | - | - |
| **Eumulgin® B2** Ceteareth-20 | - | - | - | 1,0 | - | - | - | - | - | - |
| **Lameform® TGI** Polyglyceryl-3 Isostearate | - | - | - | 4,0 | - | - | - | - | - | - |
| **Dehymuls®** PGPH Polyglyceryl-2 Dipolyhydroxystearate | - | - | 1,0 | - | - | - | - | - | - | - |
| **Monomuls® 90-L 12** Glyceryl Laurate | - | - | - | - | - | - | - | - | 1,0 | 1,0 |
| **Cetiol® HE** PEG-7 Glyceryl Cocoate | - | 0,2 | - | - | - | - | - | - | - | - |
| **Eutanol® G** Octyldodecanol | - | - | - | 3,0 | - | - | - | - | - | - |
| **Nutrilan® Keratin W** Hydrolyzed Keratin | - | - | - | - | - | - | - | - | 2,0 | 2,0 |
| **Nutrilan® I** Hydrolyzed Collagen | 1,0 | - | - | - | - | 2,0 | - | 2,0 | - | - |
| **Lamesoft® LMG** Glyceryl Laurate (and) Potassium Cocoyl Hydrolyzed Collagen | - | - | - | - | - | - | - | - | 1,0 | - |
| **Lamesoft® 156** Hydrogenated Tallow Gyceride (and) Potassium Cocoyl Hydrolyzed Collagen | - | - | - | - | - | - | - | - | - | 5,0 |
| **Gluadin® WK** Sodium Cocoyl Hydrolyzed Wheat Protein | 1,0 | 1,5 | 4,0 | 1,0 | 3,0 | 1,0 | 2,0 | 2,0 | 2,0 | - |
| **Euperlan® PK 3000 AM** Glycol Distearate (and) Laureth-4 (and) Cocamidopropyl Betaine | 5,0 | 3,0 | 4,0 | - | - | - | - | 3,0 | 3,0 | - |
| **Arlypon® F** Laureth-2 | 2,6 | 1,6 | - | 1,0 | 1,5 | - | - | - | - | - |
| **Mikrokapseln nach Beispiel 4** | 1,0 | 1,0 | 1,0 | 1,0 | 1,0 | 1,0 | 1,0 | 1,0 | 1,0 | 1,0 |
| **Hydagen® CMF** Chitosan | 1,0 | 1,0 | 1,0 | 1,0 | 1,0 | 1,0 | 1,0 | 1,0 | 1,0 | 1,0 |
| **Sodium Chloride** | - | - | - | - | - | 1,6 | 2,0 | 2,2 | - | 3,0 |
| **Glycerin** (86 Gew.-%ig) | - | 5,0 | - | - | - | - | - | 1,0 | 3,0 | - |

| **Zusammensetzung (INCI)** | **21** | **22** | **23** | **24** | **25** | **26** | **27** | **28** | **29** | **30** |
|---|---|---|---|---|---|---|---|---|---|---|
| **Texapon® NSO** Sodium Laureth Sulfate | - | 30,0 | 30,0 | - | 25,0 | - | - | - | - | - |
| **Plantacare® 818** Coco Glucosides | - | 10,0 | - | - | 20,0 | - | - | - | - | - |
| **Plantacare® PS 10** Sodium Laureth Sulfate (and) Coco Glucosides | 22,0 | - | 5,0 | 22,0 | - | - | - | - | - | - |
| **Dehyton® PK 45** Cocamidopropyl Betaine | 15,0 | 10,0 | 15,0 | 15,0 | 20,0 | - | - | - | - | - |
| **Emulgade® SE** Glyceryl Sterate (and) Ceteareth 12/20 (and) Cetearyl Alcohol (and) Cetyl Palmitate | - | - | - | - | - | 5,0 | 5,0 | 4,0 | - | - |
| **Eumulgin® B1** Ceteareth-12 | - | - | - | - | - | - | - | 1,0 | - | - |
| **Lameform® TGI** Polyglyceryl-3 Isostearate | - | - | - | - | - | - | - | - | 4,0 | - |
| **Dehymuls® PGPH** Polyglyceryl-2 Dipolyhydroxystearate | - | - | - | - | - | - | - | - | - | 4,0 |
| **Monomuls® 90-O** 18 Glyceryl Oleate | - | - | - | - | - | - | - | - | 2,0 | - |
| **Cetiol® HE** PEG-7 Glyceryl Cocoate | 2,0 | - | - | 2,0 | 5,0 | - | - | - | - | 2,0 |
| **Cetiol® OE** Dicaprylyl Ether | - | - | - | - | - | - | - | - | 5,0 | 6,0 |
| **Cetiol® PGL** Hexyldecanol (and) Hexyldecyl Laurate | - | - | - | - | - | - | - | 3,0 | 10,0 | 9,0 |
| **Cetiol® SN** Cetearyl Isononanoate | - | - | - | - | - | 3,0 | 3,0 | - | - | - |
| **Cetiol® V** Decyl Oleate | - | - | - | - | - | 3,0 | 3,0 | - | - | - |
| **Myritol® 318** Coco Caprylate Caprate | - | - | - | - | - | - | - | 3,0 | 5,0 | 5,0 |
| **Bees Wax** | - | - | - | - | - | - | - | - | 7,0 | 5,0 |
| **Nutrilan® Elastin E20** Hydrolyzed Elastin | - | - | - | - | - | 2,0 | - | - | - | - |
| **Nutrilan® I-50** Hydrolyzed Collagen | - | - | - | - | 2,0 | - | 2,0 | - | - | - |
| **Gluadin® AGP** Hydrolyzed Wheat Gluten | 0,5 | 0,5 | 0,5 | - | - | - | - | 0,5 | - | - |
| **Gluadin® WK** Sodium Cocoyl Hydrolyzed Wheat Protein | 2,0 | 2,0 | 2,0 | 2,0 | 5,0 | - | - | - | 0,5 | 0,5 |
| **Euperlan® PK 3000 AM** Glycol Distearate (and) Laureth-4 (and) Cocamidopropyl Betaine | 5,0 | - | - | 5,0 | - | - | - | - | - | - |
| **Arlypon® F** Laureth-2 | - | - | - | - | - | - | - | - | - | - |
| **Mikrokapseln nach Beispiel 4** | 1,0 | 1,0 | 1,0 | 1,0 | 1,0 | 1,0 | 1,0 | 1,0 | 1,0 | 1,0 |
| **Hydagen® CMF** Chitosan | 1,0 | 1,0 | 1,0 | 1,0 | 1,0 | 1,0 | 1,0 | 1,0 | 1,0 | 1,0 |
| **Magnesium Sulfate Hepta Hydrate** | - | - | - | - | - | - | - | - | 1,0 | 1,0 |
| **Glycerin** (86 Gew.-%ig) | - | - | - | - | - | 3,0 | 3,0 | 5,0 | 5,0 | 3,0 |

| **Zusammensetzung (INCI)** | **31** | **32** | **33** | **34** | **35** | **36** | **37** | **38** | **39** | **40** |
|---|---|---|---|---|---|---|---|---|---|---|
| **Dehymuls® PGPH** Polyglyceryl-2 Dipolyhydroxystearate | 4,0 | 3,0 | - | 5,0 | - | - | - | - | - | - |
| **Lameform® TGI** Polyglyceryl-3 Diisostearate | 2,0 | 1,0 | - | - | - | - | - | - | - | - |
| **Emulgade® PL 68/50** Cetearyl Glucoside (and) Cetearyl Alcohol | - | - | - | - | 4,0 | - | - | - | 3,0 | - |
| **Eumulgin®B2** Ceteareth-20 | - | - | - | - | - | - | - | 2,0 | - | - |
| **Tegocare® PS** Polyglyceryl-3 Methylglucose Distearate | - | - | 3,0 | - | - | - | 4,0 | - | - | - |
| **Eumulgin VL 75** Polyglyceryl-2 Dipolyhydroxystearate (and) Lauryl Glucoside (and) Glycerin | - | - | - | - | - | 3,5 | - | - | 2,5 | - |
| **Bees Wax** | 3,0 | 2,0 | 5,0 | 2,0 | - | - | - | - | - | - |
| **Cutina® GMS** Glyceryl Stearate | - | - | - | - | - | 2,0 | 4,0 | - | - | 4,0 |
| **Lanette® O** Cetearyl Alcohol | - | - | 2,0 | - | 2,0 | 4,0 | 2,0 | 4,0 | 4,0 | 1,0 |
| **Antaron® V 216** PVP / Hexadecene Copolymer | - | - | - | - | - | 3,0 | - | - | - | 2,0 |
| **Myritol® 818** Cocoglycerides | 5,0 | - | 10,0 | - | 8,0 | 6,0 | 6,0 | - | 5,0 | 5,0 |
| **Finsolv® TN** C12/15 Alkyl Benzoate | - | 6,0 | - | 2,0 | - | - | 3,0 | - | - | 2,0 |
| **Cetiol® J 600** Oleyl Erucate | 7,0 | 4,0 | 3,0 | 5,0 | 4,0 | 3,0 | 3,0 | - | 5,0 | 4,0 |
| **Cetiol® OE** Dicaprylyl Ether | 3,0 | - | 6,0 | 8,0 | 6,0 | 5,0 | 4,0 | 3,0 | 4,0 | 6,0 |
| **Mineral Oil** | - | 4,0 | - | 4,0 | - | 2,0 | - | 1,0 | - | - |
| **Cetiol® PGL** Hexadecanol (and) Hexyldecyl Laurate | - | 7,0 | 3,0 | 7,0 | 4,0 | - | - | - | 1,0 | - |
| **Bisabolol** | 1,2 | 1,2 | 1,2 | 1,2 | 1,2 | 1,2 | 1,2 | 1,2 | 1,2 | 1,2 |
| **Mikrokapseln nach Beispiel 4** | 1,0 | 1,0 | 1,0 | 1,0 | 1,0 | 1,0 | 1,0 | 1,0 | 1,0 | 1,0 |
| **Hydagen® CMF** Chitosan | 1,0 | 1,0 | 1,0 | 1,0 | 1,0 | 1,0 | 1,0 | 1,0 | 1,0 | 1,0 |
| **Copherol® F 1300** Tocopherol / Tocopheyl Acetate | 0,5 | 1,0 | 1,0 | 2,0 | 1,0 | 1,0 | 1,0 | 2,0 | 0,5 | 2,0 |
| **Neo Heliopan® Hydro** Sodium Phenylbenzimidazole Sulfonate | 3,0 | - | - | 3,0 | - | - | 2,0 | - | 2,0 | - |
| **Neo Heliopan® 303** Octocrylene | - | 5,0 | - | - | - | 4,0 | 5,0 | - | - | 10,0 |
| **Neo Heliopan® BB** Benzophenone-3 | 1,5 | - | - | 2,0 | 1,5 | - | - | - | 2,0 | - |
| **Neo Heliopan® E 1000** Isoamyl p-Methoxycinnamate | 5,0 | - | 4,0 | - | 2,0 | 2,0 | 4,0 | 10,0 | - | - |
| **Neo Heliopan® AV** Octyl Methoxycinnamate | 4,0 | - | 4,0 | 3,0 | 2,0 | 3,0 | 4,0 | - | 10,0 | 2,0 |
| **Uvinul® T 150** Octyl Triazone | 2,0 | 4,0 | 3,0 | 1,0 | 1,0 | 1,0 | 4,0 | 3,0 | 3,0 | 3,0 |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |

| | | | | | | | | | | |
|---|---|---|---|---|---|---|---|---|---|---|
| (1-4) Haarspülung, (5-6) Haarkur, (7-8) Duschbad, (9) Duschgel, (10) Waschlotion (11-14) Duschbad "Two-in-One), (15-20) Shampoo (21-25) Schaumbad, (26) Softcreme, (27, 28) Feuchtigkeitsemulsion, (29, 30) Nachtcreme (31) W/O-Sonnenschutzcreme, (32-34) W/O-Sonnenschutzlotion, (35, 38, 40) O/W-Sonnenschutzlotion (36, 37, 39) O/W-Sonnenschutzcreme | | | | | | | | | | |

## Patentansprüche

1. Mikrokapseln mit mittleren Durchmessern im Bereich von 0,001 bis 0,5 mm, bestehend aus einer doppelten Hüllmembran und einer die Wirkstoffe enthaltenden Matrix, dadurch erhältlich, dass man
(a) eine Lösung mindestens eines Wirkstoffs in einem geeigneten Lösungsmittel herstellt,
(b) die Lösung mit einem Polysaccharid versetzt,
(c) die so erhaltene Matrix bei pH-Werten im Bereich von 6 bis 8 mit einer Chitosanlösung versetzt,
(d) die so erhaltenen Mikrokapseln vom Lösungsmittel befreit und
(e) mit einer Lösung eines filmbildenden nichtionischen Polymers behandelt.

2. Verfahren zur Herstellung von Mikrokapseln mit mittleren Durchmessern im Bereich von 0,001 bis 0,5 mm, bestehend aus einer doppelten Hüllmembran und einer die Wirkstoffe enthaltenden Matrix, bei dem man
(a) eine Lösung mindestens eines Wirkstoffs in einem geeigneten Lösungsmittel herstellt,
(b) die Lösung mit einem Polysaccharid versetzt,
(c) die so erhaltene Matrix bei pH-Werten im Bereich von 6 bis 8 mit einer Chitosanlösung versetzt,
(d) die so erhaltenen Mikrokapseln vom Lösungsmittel befreit und
(e) mit einer Lösung eines filmbildenden nichtionischen Polymers behandelt.

3. Verfahren nach Anspruch 2, **dadurch gekennzeichnet, dass** man Wirkstoffe einsetzt, die ausgewählt sind aus der Gruppe, die gebildet wird von Tocopherol, Tocopherolacetat, Tocopherolpalmitat, Ascorbinsäure, (Desoxy)Ribonucleinsäuren, Retinol, Bisabolol, Allantoin, Phytantriol, Panthenol, AHA-Säuren, Kojisäure, Lipoinsäure, Aminosäuren, Ceramiden, Pseudoceramiden, essentiellen Ölen, Pflanzenextrakten und Vitaminkomplexen sowie deren Gemischen.

4. Verfahren nach den Ansprüchen 2 und 3, **dadurch gekennzeichnet, dass** man als Polysaccharide Stärke, Cellulose oder deren Derivate einsetzt.

5. Verfahren nach mindestens einem der Ansprüche 2 bis 4, **dadurch gekennzeichnet, dass** man als Lösungsmittel Wasser, niedere aliphatische Alkohole oder deren Gemische einsetzt.

6. Verfahren nach mindestens einem der Ansprüche 2 bis 5, **dadurch gekennzeichnet, dass** man Chitosane einsetzt, die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen

7. Verfahren nach mindestens einem der Ansprüche 2 bis 6, **dadurch gekennzeichnet, dass** man als nichtionisches filmbildendes Polymer Polyvinylpyrrolidon einsetzt.

8. Verwendung von Mikrokapseln nach Anspruch 1 zur Herstellung von kosmetischen und/oder pharmazeutischen Zubereitungen

9. Verwendung von Mikrokapseln nach Anspruch 1 zur Herstellung von Nahrungsmittelergänzungsstoffen.

10. Verwendung von Mikrokapseln nach Anspruch 1 zur Herstellung von Mitteln für die Textiltechnik oder Landwirtschaft.

## Claims

1. Microcapsules with mean diameters of 0.001 to 0.5 mm consisting of a double membrane and a matrix containing the active components, **characterized in that** they are obtainable by
(a) preparing a solution of at least one active component in a suitable solvent,
(b) adding a polysaccharide to the solution,
(c) adding a chitosan solution to the matrix thus obtained at pH values of 6 to 8,
(d) freeing the microcapsules thus obtained from the solvent and
(e) treating them with a solution of a film-forming polymer.

2. A process for the production of microcapsules with mean diameters of 0.001 to 0.5 mm consisting of a double membrane and a matrix containing the active components, **characterized in that** it comprises the steps of
(a) preparing a solution of at least one active component in a suitable solvent,
(b) adding a polysaccharide to the solution,
(c) adding a chitosan solution to the matrix thus obtained at pH values of 6 to 8,
(d) freeing the microcapsules thus obtained from the solvent and
(e) treating them with a solution of a film-forming polymer.

3. A process as claimed in claim 2, **characterized in that** the active components used are selected from the group consisting of tocopherol, tocopherol acetate, tocopherol palmitate, ascorbic acid, (deoxy)ribonucleic acids, retinol, bisabolol, allantoin, phytantriol, panthenol, AHA acids, koji acid, liponic acid, amino acids, ceramides, pseudoceramides, essential oils, plant extracts and vitamin complexes and mixtures thereof.

4. A process as claimed in claims 2 and 3, **characterized in that** starch, cellulose or derivatives thereof are used as the polysaccharides.

5. A process as claimed in at least one of claims 2 to 4, **characterized in that** water, lower aliphatic alcohols or mixtures thereof are used as the solvents.

6. A process as claimed in at least one of claims 2 to 5, **characterized in that** the chitosans used have an average molecular weight of 10,000 to 500,000 or 800,000 to 1,200,000 dalton and/or a Brookfield viscosity (1% by weight in glycolic acid) below 5,000 mPas, a degree of deacetylation of 80 to 88% and an ash content of less than 0.3% by weight.

7. A process as claimed in at least one of claims 2 to 6, **characterized in that** polyvinyl pyrrolidone is used as the nonionic film-forming polymer.

8. Use of the microcapsules claimed in claim 1 for the production of cosmetic and/or pharmaceutical preparations.

9. Use of the microcapsules claimed in claim 1 for the production of food products.

10. Use of the microcapsules claimed in claim 1 for the production of preparations for the textile industry or agriculture.

## Revendications

1. Microcapsules ayant des diamètres moyens dans la gamme de 0,001 à 0,5 mm, constituées d'une double membrane enveloppante et d'une matrice contenant les substances actives, pouvant être obtenue en
(a) préparant une solution d'au moins une substance active dans un solvant approprié,
(b) mélangeant cette solution avec un polysaccharide,
(c) mélangeant la matrice ainsi obtenue avec une solution de chitosane à des pH dans la gamme de 6 à 8,
(d) libérant les microcapsules ainsi obtenues du solvant, et
(e) traitant celles-ci avec une solution d'un polymère filmogène non ionique.

2. Procédé de préparation de microcapsules ayant des diamètres moyens dans la gamme de 0,001 à 0,5 mm, constituées d'une double membrane enveloppante et d'une matrice contenant les substances actives, selon lequel :
(a) on prépare une solution d'au moins une substance active dans un solvant approprié,
(b) on mélange cette solution avec un polysaccharide,
(c) on mélange la matrice ainsi obtenue avec une solution de chitosane à des pH dans la gamme de 6 à 8,
(d) on libère les microcapsules ainsi obtenues du solvant et
(e) on traite celles-ci avec une solution d'un polymère filmogène non ionique.

3. Procédé selon la revendication 2,
**caractérisé en ce qu'**
on utilise des substances actives choisies dans le groupe constitué par le tocophérol, l'acétate de tocophérol, le palmitate de tocophérol, l'acide ascorbique, les acides (désoxy)ribonucléiques, le rétinol, le bisabolol, l'allantoïne, le phytantriol, le panthénol, les acides alpha hydroxylés, l'acide kojique, l'acide lipoïdique, les acides aminés, les céramides, les pseudocéramides, les huiles essentielles, les extraits végétaux et les complexes vitaminiques ainsi que leurs mélanges.

4. Procédé selon les revendications 2 et 3,
**caractérisé en ce qu'**
on utilise comme polysaccharides de l'amidon, de la cellulose ou leurs dérivés.

5. Procédé selon au moins l'une des revendications 2 à 4,
**caractérisé en ce que**
comme solvant on utilise de l'eau, des alcools aliphatiques inférieurs ou leurs mélanges.

6. Procédé selon au moins l'une des revendications 2 à 5,
**caractérisé en ce qu'**
on utilise des chitosanes qui ont un poids moléculaire moyen de 10 000 à 500 000 ou de 800 000 à 1 200 000 dalton et/ou présentent une viscosité selon Brookfield (1 % dans de l'acide glycolique) inférieure à 5 000 mPas, un degré de désacétylation dans la gamme de 80 à 88 % et une teneur en cendres inférieure à 0,3 % en poids.

7. Procédé selon au moins l'une des revendications 2 à 6,
**caractérisé en ce qu'**
comme polymère filmogène non ionique on utilise de la polyvinylpyrrolidone.

8. Utilisation de microcapsules selon la revendication 1, pour l'obtention de préparations cosmétiques et/ou pharmaceutiques.

9. Utilisation de microcapsules selon la revendication 1, pour l'obtention de compléments alimentaires.

10. Utilisation de microcapsules selon la revendication 1, pour la l'obtention de produits pour l'industrie textile ou l'agriculture.
